# EUROPÄISCHE PATENTANMELDUNG

(11) **EP 0 658 535 A1**
(43) Veröffentlichungstag der Anmeldung: **21.06.1995**
(21) Anmeldenummer: 94203515.5
(22) Anmeldetag: 05.12.1994
(51) Int. Cl.: C07C 67/08, C07C 69/24

(54) **Verfahren zum Erzeugen von Wachsester**

(30) Priorität: 18.12.1993 DE 4343320
(71) Anmelder: METALLGESELLSCHAFT AG, D-60323 Frankfurt am Main (DE)
(72) Erfinder: Schlichting, Eberhard Dr., D-61273 Wehrheim (DE); Buchold, Henning Dr., D-63452 Hanau (DE); Mallok, Gerd, D-63452 Hanau (DE); Gärtner, Fritz-Jürgen Dr., D-64404 Bickenbach (DE); Stönner, Hans-Martin, D-65760 Eschborn (DE)

(57) **Zusammenfassung**

Der Wachsester wird aus einem mindestens eine Fettsäure und mindestens einen Fettalkohol enthaltenden flüssigen Gemisch erzeugt, wobei die Fettsäure und der Fettalkohol pro Molekül 6 bis 30 C-Atome enthalten. Das Gemisch wird bei Temperaturen im Bereich von etwa 105 bis 300°C bewegt und in eine Sprühzone hinein versprüht. In der Sprühzone entsteht eine wasserdampfhaltige Atmosphäre, die man mindestens teilweise entfernt. Vor der Sprühzone kann das Gemisch aus Fettsäure und Fettalkohol in mindestens einem Rührkessel bewegt werden. Vorzugsweise wird ohne Katalysatorzugabe gearbeitet.

## Beschreibung

Die Erfindung betrifft ein verfahren zum Erzeugen von Wachsester aus einem mindestens eine Fettsäure und mindestens einen Fettalkohol enthaltenden flüssigen Gemisch, wobei die Fettsäure und der Fettalkohol pro Molekül 6 bis 30 C-Atome enthalten.

In der Zeitschrift JAOCS, Band 69, Nr. 11 (November 1992), Seite 1150 bis 1153 wird ein Verfahren dieser Art beschrieben, wobei man das Fettsäure-Fettalkohol-Gemisch zusammen mit einem Zeolith-Katalysator bei Temperaturen im Bereich von 150 bis 275°C in einer Laborapparatur rührt.

Der Erfindung liegt die Aufgabe zugrunde, das eingangs genannte Verfahren auf einfache Weise großtechnisch durchzuführen. Erfindungsgemäß geschieht dies dadurch, daß das Gemisch bei Temperaturen im Bereich von etwa 105 bis 300°C bewegt und in eine Sprühzone hinein versprüht wird, wobei in der Sprühzone eine wasserdampfhaltige Atmosphäre erzeugt wird, die man mindestens teilweise entfernt. Das Bewegen und dabei intensive Mischen der Reaktionsteilnehmer kann z.B. durch Rühren oder Umwälzen erfolgen, wobei sich das Umwälzen z.B. durch die Strömungsführung und/oder durch Inertgaszufuhr mit beträchtlicher Turbulenz durchführen läßt.

Auch wenn die Zugabe eines Katalysators verfahrensbeschleunigend wirken kann, so läßt sich das Verfahren der Erfindung doch auch ohne Katalysator durchführen. Da der Verzicht auf einen Katalysator bei der großtechnischen Wachsester-Herstellung erhebliche Kostenvorteile bringt, wird man eine solche Verfahrensweise bevorzugen.

Insbesondere wenn ohne Katalysator gearbeitet wird, ist es Zweckmäßig, das Gemisch während des Bewegens und des Versprühens auf Temperaturen von mindestens 120°C zu halten.

Das Verfahren kann kontinuierlich oder chargenweise durchgeführt werden. Für die kontinuierliche Arbeitsweise empfiehlt es sich, die Fettsäure und den Fettalkohol in einer ersten Reaktionszone zu mischen, wobei ein wachsesterhaltiges Gemisch entsteht, das man durch mindestens eine weitere Reaktionszone leitet, in welcher man das Gemisch bewegt, und daß man das Gemisch in mindestens einer Sprühzone versprüht.

Beim erfindungsgemäßen Verfahren ist es wichtig, daß der bei der Veresterung entstehende Wasserdampf mindestens teilweise aus dem Reaktionsgemisch entfernt wird. Dies kann auf verschiedene Weise erfolgen, zum Beispiel dadurch, daß man die wasserdampfhaltige Atmosphäre über dem Gemisch absaugt. Eine andere Möglichkeit besteht darin, durch das Gemisch ein Inertgas, zum Beispiel Stickstoff, hindurchzuleiten. Das Inertgas erzeugt einen Strippeffekt, wobei das Inertgas Wassermoleküle sowohl aus dem flüssigen Gemisch als auch aus der Sprühzone mitnimmt.

Es empfiehlt sich, das Gemisch in die Sprühzone hinein fein zu verdüsen, wobei man Tröpfchendurchmesser vorzugsweise im Bereich von 0,01 bis 5,0 mm ausbildet.

Das Verhältnis von Fettsäure zu Fettalkohol zu Beginn der Umsetzung kann in weitem Bereich variieren. Wenn man ein wachsesterreiches Produkt erzeugen will, das nur noch geringe Reste an Fettsäure und Fettalkohol aufweist, wird man Fettsäure und Fettalkohol im Molverhältnis 1:1 vorlegen. Wenn es darauf ankommt, ein möglichst säurefreies Wachsester-Produkt herzustellen, empfiehlt es sich, mit einem Überschuß an Fettalkohol zu beginnen. Auf diese Weise ist es ohne weiteres möglich, Wachsester mit einer Säurezahl kleiner 1 oder auch kleiner 0,3 herzustellen. Die Säurezahl wird wie üblich gemessen in mg KOH zum Neutralisieren der restlichen Säure pro Gramm der untersuchten Probe.

Ausgestaltungsmöglichkeiten des Verfahrens werden mit Hilfe der Zeichnung erläutert. Es zeigt:
Fig. 1 eine einstufige Verfahrensweise, die besonders für chargenweisen Betrieb geeignet ist und
Fig. 2 eine dreistufige Verfahrensweise, die sich besonders für kontinuierliches Arbeiten eignet.

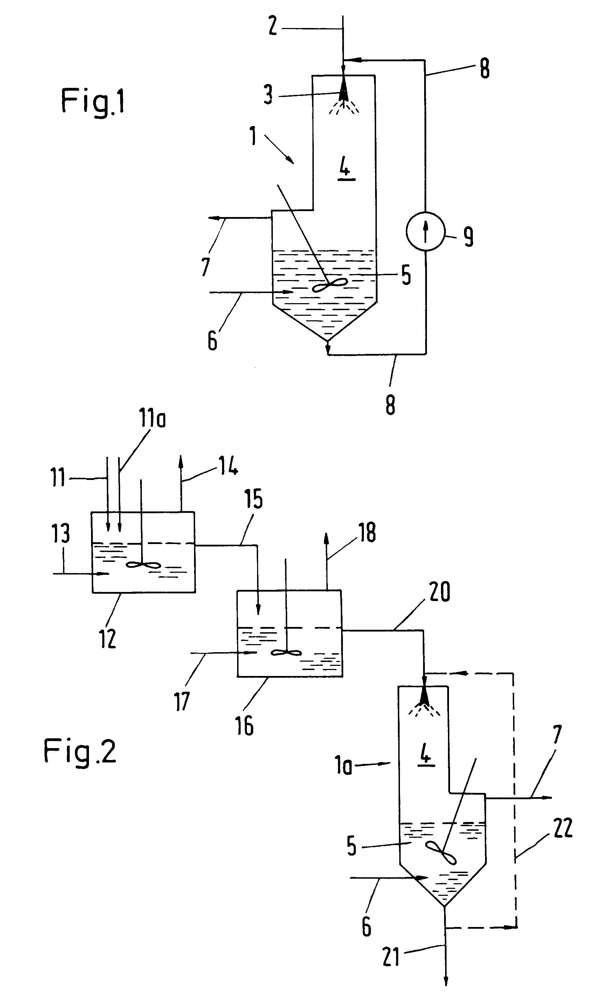

Beim Verfahren der Fig. 1 werden einem Reaktor (1) durch die Leitung (2) gemeinsam Fettsäure und Fettalkohol zugeführt und das Gemisch wird durch eine Düse (3) in feine Tröpfchen zerteilt in die Sprühzone (4) des Reaktors (1) hinein versprüht. Unterhalb der Sprühzone (4) befindet sich im Reaktor (1) eine Reaktionszone (5), wo das Gemisch aus Fettsäure, Fettalkohol und gebildetem Wachsester ständig intensiv gerührt wird. In das Gemisch wird durch die Leitung (6) Inertgas, zum Beispiel Stickstoff, eingeleitet. Bei der Umsetzung von Fettsäure mit Fettalkohol bei den im Reaktor herrschenden Temperaturen im Bereich von 105 bis 300°C und vorzugsweise von mindestens 120°C entsteht Wasserdampf, der vor allem durch das Versprühen wirksam aus dem Reaktionsgemisch abgetrennt wird. Der gebildete wasserdampf wird zusammen mit Inertgas durch die Leitung (7) aus dem Reaktor (1) abgeführt, z.B. durch Absaugen. Falls Ausgangsmaterial und Wachsester-Produkt mitgerissen wird, kann man das in an sich bekannter, nicht dargestellter Weise durch Kondensation wiedergewinnen und zurück in den Reaktor (1) leiten.

Die Ausgangsmaterial und Wachsester enthaltende Flüssigkeit, die sich in der Reaktionszone (5) sammelt, wird für eine gewisse Zeit im Kreis geführt und durch die Leitung (8) unter der Wirkung der Pumpe (9) auf den Kopf des Reaktors (1) gegeben und erneut durch die Düse (3) versprüht. Bei chargenweisem Betrieb des Reaktors (1) liegen die Verweilzeiten im Reaktor bei 1 bis 10 Stunden, wobei die Gesamtmenge der Flüssigkeit 1 bis 20 Mal/h versprüht wird.

Ebenso wie beim Verfahren der Fig. 1 kann man auch beim Verfahren der Fig. 2 ohne Katalysator arbeiten. Gemäß Fig. 2 gibt man durch die Leitung (11) Fettsäure und durch die Leitung (11a) Fettalkohol in einen ersten Reaktionsbehälter (12), in welchem das Gemisch intensiv gerührt wird. Während des Rührens führt man durch die Leitung (13) Inertgas, z.B. Stickstoff, zu. Wasserdampfhaltiges Inertgas zieht man durch die Leitung (14) ab. Im ersten Reaktionsbehälter (12) erfolgt die Umsetzung nur teilweise, so daß man in der Leitung (15) eine Fettsäure, Fettalkohol und Wachsester enthaltende Flüssigkeit abführt, die man einem zweiten Reaktionsbehälter (16) aufgibt. Dieser zweite Behälter (16) arbeitet im Prinzip genauso wie der erste Reaktionsbehälter (12), er weist ebenfalls eine Inertgaszufuhr (17) und eine Ableitung (18) für wasserdampfhaltiges Gas auf.

In der Leitung (20) zieht man aus dem zweiten Reaktionsbehälter (16) eine Flüssigkeit ab, die gegenüber der Flüssigkeit der Leitung (15) einen erhöhten Anteil an Wachsester und daneben noch Reste an Fettsäure und Fettalkohol enthält. Diese Flüssigkeit gibt man einem Reaktor (1a) auf, der, wie bereits zusammen mit Fig. 1 erläutert, eine Sprühzone (4) und eine Reaktionszone (5) aufweist. Inertgas wird durch die Leitung (6) zugeführt und wasserdampfhaltiges Gas führt man in der Leitung (7) ab. Wachsesterreiches Produkt fällt in der Leitung (21) an. Falls erforderlich, kann man den Reaktor (1a) ebenfalls mit einem Flüssigkeitskreislauf betreiben, wobei man einen Teil der Flüssigkeit der Leitung (21) durch die gestrichelt eingezeichnete Leitung (22) zum Kopf des Reaktors (1a) zurückführt. Die Gesamt-Verweilzeit beim kontinuierlichen Verfahren gemäß Fig. 2 ist üblicherweise länger als die Verweilzeit beim chargenweisen Verfahren der Fig. 1.

Das Verfahren kann abweichend von Fig. 2 auch nur mit einem Reaktionsbehälter oder aber auch mit mehr als 2 Reaktionsbehältern betrieben werden. Auch kann man mehr als einen mit Sprüheinrichtung versehenen Reaktor einsetzen.

### Beispiel 1

Im Labormaßstab wird gemäß Fig. 1 chargenweise gearbeitet und ein Reaktor (1) mit elektrisch beheizbarem Mantel verwendet. 208 g eines Fettsäuregemisches (c₈ - c₁₈) mit mittlerem Molekulargewicht der Fettsäuren von 208 wird mit 1,1 mol C₁₆-Fettalkohol dem Reaktor aufgegeben, wobei die Reaktionsteilnehmer eine Temperatur von 65°C aufweisen. Die Säurezahl des Gemisches beträgt 117 mg KOH/g. Im Reaktor herrscht eine Temperatur von 250°C, als Inertgas wird durch die Leitung (6) Stickstoff zugeführt. Pro Stunde werden 6 l Flüssigkeit durch die Düse (3) mit Tröpfchengrößen im Bereich von 0,01 bis 1,0 mm in die Sprühzone (4) hinein versprüht; bei einem Vergleichsversuch wird auf dieses Versprühen und die Leitung (8) verzichtet. Das Fortschreiten der Veresterungsreaktion wird stündlich durch Messen der Säurezahl überwacht. Ergebnisse:

| **Versuchsdauer (Stunden)** | **0** | **1** | **2** | **3** | **4** | **5** | **6** |
|---|---|---|---|---|---|---|---|
| Säurezahl **mit** Versprühen | 117 | 7,7 | 0,49 | 0,12 | | | |
| Säurezahl **ohne** Versprühen | 117 | 11,4 | 6,4 | 4,2 | 3,1 | 2,33 | 1,68 |

Beim erfindungsgemäßen Arbeiten mit Versprühen kann der Versuch nach 3 Stunden mit praktisch vollständiger Veresterung beendet werden; der Vergleichsversuch ohne Versprühen wird nach 6 Stunden ständigen Rührens des Reaktionsgemisches mit unbefriedigendem Ergebnis abgebrochen.

### Beispiel 2:

Im Labormaßstab wird gemäß Fig. 2 kontinuierlich gearbeitet, wobei man 3 Rührkessel (12), (16) und (1a) mit elektrisch beheizbarem Mantel verwendet. Der Kessel (1a) der Endstufe ist mit einer Sprühzone (4) und einer Kreislaufleitung (22) ausgestattet. Dem ersten Rührkessel (12) führt man durch die Leitung (11) das bereits für das Beispiel 1 verwendete Fettsäuregemisch (C₈-C₁₈) und durch die Leitung (11a) C₁₂-Fettalkohol, beide vorgewärmt auf 200°C zu, und zwar 1,2 mol Fettalkohol pro mol Fettsäuregemisch. In den Rührkesseln wird jeweils eine Temperatur von 250°C aufrechterhalten und Stickstoff als Inertgas zugeführt. Die Verweilzeit in den Rührkesseln beträgt 4 Stunden.

In den Leitungen (15), (20) und (21) werden die Säurezahlen 21,2; 3,8 und 0,2 gemessen, d.h. das Produkt, das man in der Leitung (21) erhält, ist praktisch säurefrei. Wenn man jedoch in der Endstufe auf die Sprühzone (4) und die Kreislaufleitung (22) verzichtet und den Reaktor (1a) nur als Rührkessel betreibt, erhält man ein Produkt mit einer Säurezahl von 2,1, d.h. der erzeugte Wachsester weist noch einen erheblichen restlichen Säuregehalt auf.

## Patentansprüche

1. Verfahren zum Erzeugen von Wachsester aus einem mindestens eine Fettsäure und mindestens einen Fettalkohol enthaltenden flüssigen Gemisch, wobei die Fettsäure und der Fettalkohol pro Molekül 6 bis 30 C-Atome enthalten, dadurch gekennzeichnet, daß das Gemisch bei Temperaturen im Bereich von etwa 105 bis 300°C bewegt und in eine Sprühzone hinein versprüht wird, wobei in der Sprühzone eine wasserdampfhaltige Atmosphäre erzeugt wird, die man mindestens teilweise entfernt.

2. Verfahren nach Anspruch 1, dadurch gekennzeichnet, daß das Gemisch während des Bewegens und des Versprühens auf Temperaturen von mindestens 120°C gehalten wird.

3. Verfahren nach Anspruch 1 oder 2, dadurch gekennzeichnet, daß die Fettsäure und der Fettalkohol in einer ersten Reaktionszone gemischt werden, wobei ein wachsesterhaltiges Gemisch entsteht, das man durch mindestens eine weitere Reaktionszone leitet, in welcher man das Gemisch bewegt, und daß man das Gemisch in mindestens einer Sprühzone versprüht.

4. Verfahren nach einem der Ansprüche 1 bis 3, dadurch gekennzeichnet, daß das Gemisch ohne Katalysatorzugabe bewegt und versprüht wird.

5. Verfahren nach einem der Ansprüche 1 bis 4, dadurch gekennzeichnet, daß man in das Gemisch während des Bewegens ein Inertgas einleitet und wasserdampfhaltiges Inertgas aus dem Gemisch ableitet.

6. Verfahren nach einem der Ansprüche 1 bis 5, dadurch gekennzeichnet, daß man den während der Reaktion entstehenden Wasserdampf oberhalb des Gemisches absaugt.

7. Verfahren nach einem der Ansprüche 1 bis 6, dadurch gekennzeichnet, daß man im Gemisch einen Überschuß an Fettalkohol aufrechterhält und ein wachsesterreiches, praktisch säurefreies Produkt erzeugt.
